# FASCICULE DE BREVET EUROPEEN

(11) **EP 1 272 454 B1**
(45) Date de publication et mention de la délivrance du brevet: **23.05.2007**
(21) Numéro de dépôt: 01923776.7
(22) Date de dépôt: 10.04.2001
(51) Int. Cl.: C07C 227/32

(54) **PROCEDE DE SYNTHESE DES ESTERS DE LA N-[(S)-1-CARBOXYBUTYL]-(S)-ALANINE ET APPLICATION A LA SYNTHESE DU PERINDOPRIL**
VERFAHREN ZUR HERSTELLUNG VON ESTERN DES N-[(S)-1-CARBOXYBUTYL]-(S)-ALANINS UND IHRE VERWENDUNG IN DER SYNTHESE VON PERINDOPRIL
METHOD FOR SYNTHESIS OF N-[ (S)-1-CARBOXYBUTYL]-(S)-ALANINE ESTERS AND USE IN SYNTHESIS OF PERINDOPRIL

(30) Priorité: 11.04.2000 FR 0004610
(43) Date de publication de la demande: 08.01.2003
(73) Titulaire: Les Laboratoires Servier, 92415 Courbevoie Cedex (FR)
(72) Inventeur: SOUVIE, Jean-Claude, F-76600 Le Havre (FR); RENAUD, Alain, F-76000 Rouen (FR)
(86) Numéro de dépôt international: PCT/FR2001/001088
(87) Numéro de publication internationale: WO 2001/056972

(56) Documents cités:
- EP-A- 0 308 340
- EP-A- 0 309 324
- M. VINCENT ET AL.: "Stereoselective Synthesis of a New Perhydroindole Derivative of Chiral Iminodiacid, a Potent Inhibitor of Agiotensin Converting Enzyme" TETRAHEDRON LETTERS, vol. 23, no. 16, 1982, pages 1677-1680, XP002155080 OXFORD GB cité dans la demande

## Description

La présente invention concerne un procédé de synthèse industrielle des esters de la N-[(S)-1-carboxybutyl]-(S)-alanine, et leur application à la synthèse industrielle du perindopril et de ses sels pharmaceutiquement acceptables.

Plus spécifiquement, la présente invention concerne un nouveau procédé de synthèse industrielle des dérivés de formule (I): 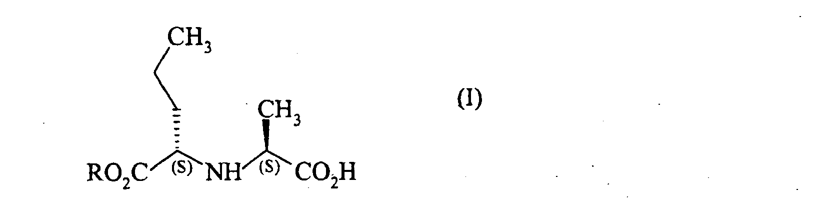 dans laquelle R représente un groupement alkyle (C₁-C₆) linéaire ou ramifié,
ainsi que leurs sels d'addition à un acide ou à une base minéral(e) ou organique.

Les composés de formule (I) obtenus selon le procédé de l'invention sont utiles dans la synthèse du perindopril de formule (II) : 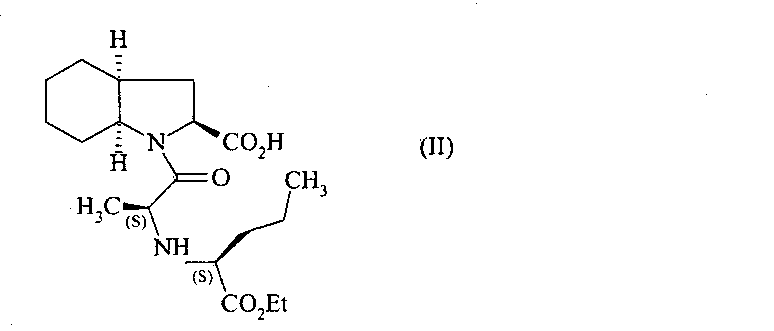 ainsi que dans celle de ses sels pharmaceutiquement acceptables.

Le perindopril, ainsi que ses sels, possèdent des propriétés pharmacologiques intéressantes. Leur principale propriété est d'inhiber l'enzyme de conversion de l'angiotensine I (ou kininase II), ce qui permet d'une part d'empêcher la transformation du décapeptide angiotensine 1 en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse industrielle performant, permettant notamment l'obtention sélective du diastéréoisomère (S,S) avec un bon rendement et une excellente pureté.

Quelques méthodes de préparation des composés de formule (I) sont déjà connues. Toutefois, ces procédés présentent des inconvénients importants à l'échelle industrielle:
- Le journal Tel. Lett. 1982, 23 (16), 1677-80 décrit l'obtention d'un dérivé de formule (I) (R = éthyle) par réaction dans l'éthanol du 2-oxovalérate d'éthyle avec l'ester tert-buylique de l'alanine en présence de cyanoborohydrure de sodium. Mais cet agent réducteur est particulièrement toxique, très hygroscopique et difficilement manipulable à l'échelle industrielle.
- Les brevets EP 0 308 340 et EP 0 308 341 décrivent l'accès à un composé de formule (I) (R = éthyle) par réaction dans l'eau du chlorhydrate de norvalinate d'éthyle avec l'acide pyruvique en présence d'hydrogène, de charbon palladié et de soude. L'isolement du produit brut se fait ensuite par évaporation de l'eau, puis de l'éthanol est ajouté pour précipiter le chlorure de sodium formé pendant la réaction. Après filtration, la solution éthanolique obtenue est évaporée et le résidu est recristallisé dans l'acétonitrile.
   Ce procédé présente plusieurs inconvénients:
   * L'acide pyruvique étant instable, son utilisation génère des impuretés dans le milieu réactionnel.
   * La réaction se fait à pH proche de la neutralité. L'utilisation du chlorhydrate de norvalinate d'éthyle nécessite donc l'ajout de soude en quantité importante (1,1 à 1,2 moles pour 1 mole de chlorhydrate de norvalinate utilisé), ce qui génère une quantité importante de chlorure de sodium, rendant laborieuse son élimination lors de l'isolement à l'échelle industrielle.
   * L'isolement comprend une étape d'évaporation de l'eau du milieu réactionnel, particulièrement longue à cette échelle.
- Le brevet EP 0 309 324 décrit l'accès à un composé de formule (I) (R = éthyle) par réaction dans le diméthylformamide de l'ester benzylique de l'alanine avec l'α-bromovalérate d'éthyle en présence de triéthylamine. Les inconvénients majeurs de ce procédé sont le nombre important d'étapes et le faible rendement en isomère (S,S). En effet, la réaction n'étant pas diastéréosélective, on est obligé, pour accéder à l'isomère (S,S) pur, de rajouter une étape de purification par cristallisation fractionnée en présence d'acide maléique.

La demanderesse a présentement mis au point un procédé de synthèse industrielle des dérivés de formule (I) très intéressant, d'une part parce qu'il permet l'obtention directe du diastéréoisomère (S,S) avec une très bonne pureté et un bon rendement, d'autre part parce que l'isolement est particulièrement rapide et simple à mettre en oeuvre à l'échelle industrielle, enfin parce qu'il utilise comme source de chiralité l'alanine, matière première naturelle et donc peu coûteuse.

Plus spécifiquement, la présente invention concerne un procédé de synthèse industrielle des composés de formule (I) caractérisé en ce que l'on condense l'alanine de formule (III): 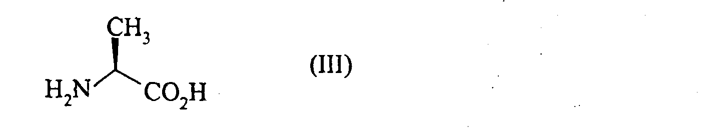 avec un composé de formule (IV): 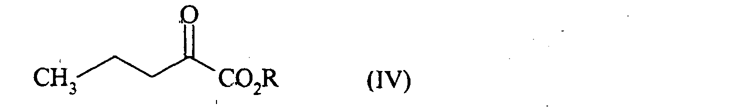 dans laquelle R a la même signification que dans la formule (I),
sous hydrogénation catalysée par le charbon palladié à 5 %,
dans l'eau,
sous une pression comprise entre 1 et 30 bars, préférentiellement entre 1 et 5 bars,
à une température comprise entre 10 et 60°C, préférentiellement entre 10 et 40°C,
en présence d'une quantité de soude comprise entre 0 et 0,5 mole, de préférence entre 0,1
et 0,2 mole par mole de dérivé de formule (IV) utilisé, pour conduire, après acidification du milieu réactionnel à pH compris entre 3 et 3,5, filtration et recristallisation du précipité obtenu dans l'acétonitrile, au dérivé de formule (I) optiquement pur.
- La faible quantité de soude utilisée dans ce procédé limite la formation de chlorure de sodium à l'acidification du milieu réactionnel, facilitant ensuite la recristallisation.
- L'isolement évite l'évaporation totale de l'eau du milieu réactionnel et est particulièrement rapide et simple à mettre en oeuvre à l'échelle industrielle.

L'exemple ci-dessous illustre l'invention, mais ne la limite en aucune façon.

### Exemple : N-[(S)-Carbéthoxy-1-butyl]-(S)-alanine

Dans une cuve équipée d'un agitateur, placer 25 kg de L-alanine en solution dans l'eau, 1,1 kg de soude et 36 kg de 2-oxo-pentanoate d'éthyle. Agiter le milieu réactionnel pendant 30 minutes, Dans un appareil à hydrogéner, placer du charbon palladié à 5 % en suspension dans l'eau, puis le mélange précédemment obtenu. Hydrogéner sous pression de 1 bar à température ambiante jusqu'à absorption de la quantité théorique d'hydrogène. Eliminer le catalyseur par filtration, puis ajouter au filtrat de l'acide chlorhydrique concentré jusqu'à pH = 3. Récolter le solide obtenu par filtration, reprendre le gâteau dans l'acétonitrile au reflux, filtrer à chaud puis laisser cristalliser.

## Revendications

1. Procédé de synthèse industrielle des composés de formule (I) 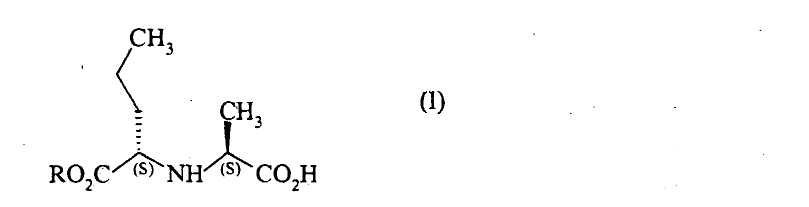 dans laquelle R représente un groupement alkyle (C₁-C₆) linéaire ou ramifié,
**caractérisé en ce que** l'on condense l'alanine de formule (III) : 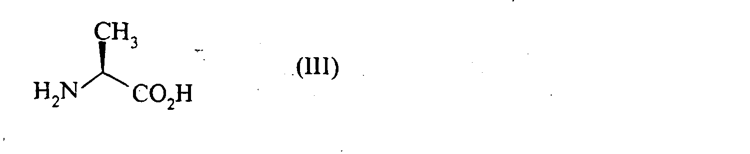 avec un composé de formule (IV) : 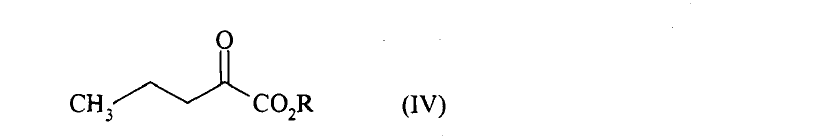 dans laquelle R a la même signification que dans la formule (I),
sous hydrogénation catalysée par le charbon palladié à 5 %,
dans l'eau,
sous une pression comprise entre 1 et 30 bars,
à une température comprise entre 10 et 60°C,
en présence d'une quantité de soude comprise entre 0 et 0,5 mole par mole de dérivé de formule (IV) utilisé, pour conduire, après acidification du milieu réactionnel à pH compris entre 3 et 3,5, filtration et recristallisation du précipité obtenu dans l'acétonitrile, au dérivé de formule (1) optiquement pur.

2. Procédé de synthèse selon la revendication 1, permettant l'obtention du dérivé de formule (I) dans laquelle R représente le groupement éthyle.

3. Procédé de synthèse selon l'une quelconque des revendications 1 ou 2, **caractérisé en ce que** la pression d'hydrogénation est comprise entre 1 et 5 bars.

4. Procédé de synthèse selon l'une quelconque des revendications 1 à 3, **caractérisé en ce que** la température d'hydrogénation est comprise entre 10 et 40°C.

5. Procédé de synthèse selon l'une quelconque des revendications 1 à 4, **caractérisé en ce que** la quantité de soude ajoutée est comprise entre 0,1 et 0,2 mole par mole de dérivé de formule (IV) utilisé.

6. Procédé de synthèse du perindopril ou de ses sels pharmaceutiquement acceptables, dans lequel l'alanine de formule (III) et le composé de formule (IV) sont mis en réaction selon le procédé de la revendication 1, puis le composé de formule (I) ainsi obtenu est transformé en perindopril ou en l'un de ses sels pharmaceutiquement acceptables.

## Claims

1. Process for the industrial synthesis of the compounds of formula (I) 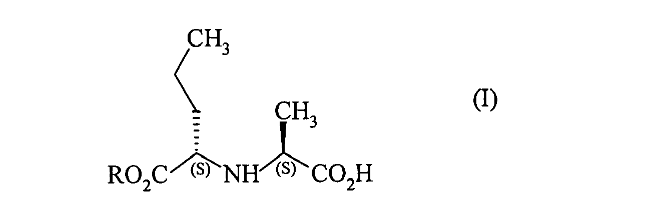 wherein R represents a linear or branched (C₁-C₆)alkyl group,
**characterised in that** alanine of formula (III) : 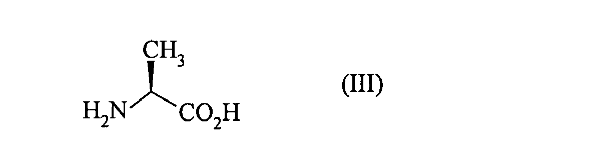 is condensed with a compound of formula (IV) : 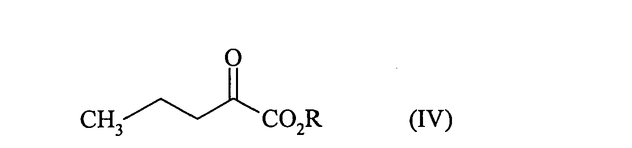 wherein R is as defined for formula (I),
with hydrogenation catalysed by 5 % palladium-on-carbon,
in water,
at a pressure of from 1 to 30 bar,
at a temperature of from 10 to 60°C,
in the presence of sodium hydroxide in an amount of from 0 to 0.5 mol per mol of compound of formula (IV) employed, in order to yield the compound of formula (I) in optically pure form following acidification of the reaction mixture to a pH of from 3 to 3.5, filtration, and recrystallisation of the resulting precipitate from acetonitrile.

2. Synthesising process according to claim 1 that allows the compound of formula (I) wherein R represents an ethyl group to be obtained.

3. Synthesising process according to either claim 1 or claim 2, **characterised in that** the hydrogenation pressure is from 1 to 5 bar.

4. Synthesising process according to any one of claims 1 to 3, **characterised in that** the hydrogenation temperature is from 10 to 40°C.

5. Synthesising process according to any one of claims 1 to 4, **characterised in that** the amount of sodium hydroxide added is from 0.1 to 0.2 mol per mol of the compound of formula (IV) employed.

6. Process for the synthesis of perindopril or a pharmaceutically acceptable salt thereof, wherein alanine of formula (III) and a compound of formula (IV) are reacted in accordance with the process according to claim 1, then the compound of formula (I) so obtained is converted into perindopril or into a pharmaceutically acceptable salt thereof.

## Patentansprüche

1. Verfahren zur technischen Synthese der Verbindungen der Formel (I): 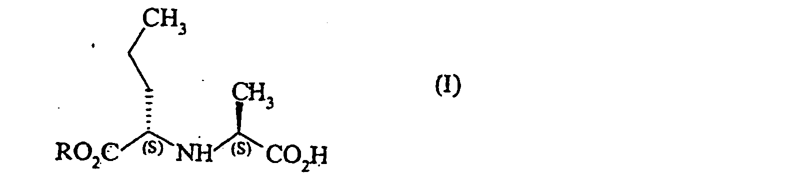 in der R eine geradkettige oder verzweigte (C₁-C₆)-Alkylgruppe bedeutet,
**dadurch gekennzeichnet, daß** man Alanin der Formel (III): 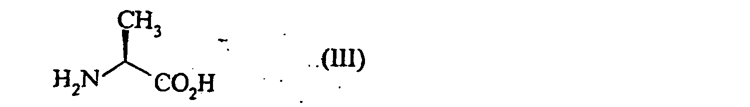 mit einer Verbindung der Formel (IV): 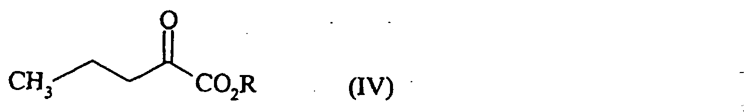 in der R die bezüglich der Formel (I) angegebenen Bedeutungen besitzt,
unter katalytischer Hydrierung mit 5 % Palladium-auf-Kohlenstoff,
in Wasser,
bei einem Druck zwischen 1 und 30 bar,
bei einer Temperatur zwischen 10 und 60 °C,
in Gegenwart einer Natriumhydroxid-Menge zwischen 0 und 0,5 Mol pro Mol des verwendeten Derivats der Formel (IV) kondensiert, so daß man nach dem Ansäuern des Reaktionsmediums auf einen pH-Wert zwischen 3 und 3,5, dem Filtrieren und dem Umkristallisieren des erhaltenen Niederschlags aus Acetonitril das Derivat der Formel (I) in optisch reiner Form erhält.

2. Syntheseverfahren nach Anspruch 1 für die Herstellung des Derivats der Formel (I), in der R die Ethylgruppe bedeutet.

3. Syntheseverfahren nach einem der Ansprüche 1 oder 2, **dadurch gekennzeichnet, daß** der Druck bei der Hydrierung zwischen 1 und 5 bar liegt.

4. Syntheseverfahren nach einem der Ansprüche 1 bis 3, **dadurch gekennzeichnet, daß** die Temperatur der Hydrierung zwischen 10 und 40 °C liegt.

5. Syntheseverfahren nach einem der Ansprüche 1 bis 4, **dadurch gekennzeichnet, daß** die zugesetzte Menge Natriumhydroxid zwischen 0.1 und 0.2 Mol pro Mol des verwendeten Derivats der Formel (IV) beträgt.

6. Verfahren zur Synthese von Perindopril oder von seinen pharmazeutisch annehmbaren Salzen, worin Alanin der Formel (III) und die Verbindung der Formel (IV) nach dem Verfahren gemäß Anspruch 1 umgesetzt werden und die in dieser Weise erhaltene Verbindung der Formel (I) in Perindopril oder eines seiner pharmazeutisch annehmbaren Salze umgewandelt wird.
